(19) Europäisches Patentamt
European Patent Office
Office européen des brevets

(11) **EP 3 671 260 B1**

(12) **EUROPEAN PATENT SPECIFICATION**

(45) Date of publication and mention
of the grant of the patent:
**03.04.2024 Bulletin 2024/14**

(21) Application number: **19159348.2**

(22) Date of filing: **26.02.2019**

(51) International Patent Classification (IPC):
*G01S 7/41* (2006.01)    *G01S 13/536* (2006.01)
*G01S 13/88* (2006.01)    *A61B 5/024* (2006.01)
*A61B 5/00* (2006.01)    *A61B 5/05* (2021.01)
*G01S 7/40* (2006.01)    *G01S 7/35* (2006.01)
*G01S 13/02* (2006.01)    *G01S 13/32* (2006.01)
*G01S 13/62* (2006.01)    *G01S 13/87* (2006.01)

(52) Cooperative Patent Classification (CPC):
**A61B 5/002; A61B 5/7282; G01S 7/354;**
**G01S 7/4021; G01S 7/414; G01S 7/415;**
**G01S 13/536; G01S 13/88;** A61B 5/02444;
A61B 5/24; G01S 7/356; G01S 7/358;
G01S 13/0209; G01S 13/32; G01S 13/62;    (Cont.)

(54) **NON-CONTACT VITAL-SIGN MONITORING SYSTEM AND METHOD**

KONTAKTLOSES VITALPARAMETERÜBERWACHUNGSSYSTEM UND -VERFAHREN

SYSTÈME ET PROCÉDÉ DE SURVEILLANCE DE SIGNES VITAUX SANS CONTACT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 TW 107146296**

(43) Date of publication of application:
**24.06.2020 Bulletin 2020/26**

(73) Proprietor: **Wistron Corporation
New Taipei City 22181 (TW)**

(72) Inventors:
• **CHIOU, Sheng-Lun
New Taipei City 22181 (TW)**
• **CHEN, Yin-Yu
New Taipei City 22181 (TW)**
• **WU, Fang-Ming
New Taipei City 22181 (TW)**
• **CHANG, Ching-Han
New Taipei City 22181 (TW)**

(74) Representative: **Karakatsanis, Georgios
Haft Karakatsanis Patent Law Office
Stadtplatz 11
94209 Regen (DE)**

(56) References cited:
**WO-A1-2007/101343**

(52) Cooperative Patent Classification (CPC): (Cont.)
G01S 13/87

**Description**

**BACKGROUND OF THE INVENTION**

**1. FIELD OF THE INVENTION**

The present invention generally relates to a monitoring system, and more particularly to a non-contact vital-sign monitoring system and method.

**2. DESCRIPTION OF RELATED ART**

Body temperature (BT), blood pressure (BP), heart rate (HR) and respiratory rate (RR) are four primary vital signs. The detection and measurement of the vital signs may be used to evaluate health condition or a clue to illness of a person.

Most conventional health monitoring devices are contact type, and contact with the body of a monitored subject via wires. Accordingly, the contact health monitoring devices would restrict the movement of the monitored subject. Moreover, the contact health monitoring devices may only be operated by trained personnel.

A professional is generally required to make observation and recording while using conventional (contact or non-contact) health monitoring devices. Due to limited manpower, health measure can only be made at intervals. Therefore, a detecting chance may probably be lost in case of emergency, and an opportunity to save life may be missed. A need has thus arisen to propose an all-day non-contact vital-sign monitoring system. WO 2007/101343 A1 discloses apparatus for monitoring vital signs of one or more living subjects comprising a monitoring station and at least one sensor in communication with the monitoring station. The sensor comprises an antenna system, an ultra wideband radar system coupled to the antenna system, a signal processor and a communication system. The signal processor is connected to receive a signal from the ultra wideband radar system and configured to extract from the signal information about one or more vital signs of a person or animal in a sensing volume corresponding to the antenna system. The communication system is configured to transmit the information to the monitoring station.

**SUMMARY OF THE INVENTION**

In view of the foregoing, it is an object of the embodiment of the present invention to provide a non-contact vital-sign monitoring system and method for detecting a respiratory rate and a heart rate.

According to one embodiment, a non-contact vital-sign monitoring system having a radar disposed in vicinity of a monitored subject includes a data buffer, a status classifier and a vital-sign detector. The data buffer stores output signals of the radar sampled in sequence during a predetermined period. The status classifier determines status of the monitored subject according to the output signals. The vital-sign detector determines a vital sign of the monitored subject according to the output signals of stationary status.

**BRIEF DESCRIPTION OF THE DRAWINGS**

FIG. 1 shows a block diagram illustrating a non-contact vital-sign monitoring system according to one embodiment of the present invention;
FIG. 2 shows a flow diagram illustrating a non-contact vital-sign monitoring method for determining vital-sign status according to one embodiment of the present invention;
FIG. 3A shows a flow diagram illustrating a method of detecting a respiratory rate according to one embodiment of the present invention;
FIG. 3B shows a flow diagram illustrating a method of detecting a heart rate according to one embodiment of the present invention;
FIG. 4 shows a flow diagram illustrating a non-contact vital-sign monitoring method according to one embodiment of the present invention; and
FIG. 5 shows a block diagram illustrating a non-contact vital-sign monitoring system according to another embodiment of the present invention.

**DETAILED DESCRIPTION OF THE INVENTION**

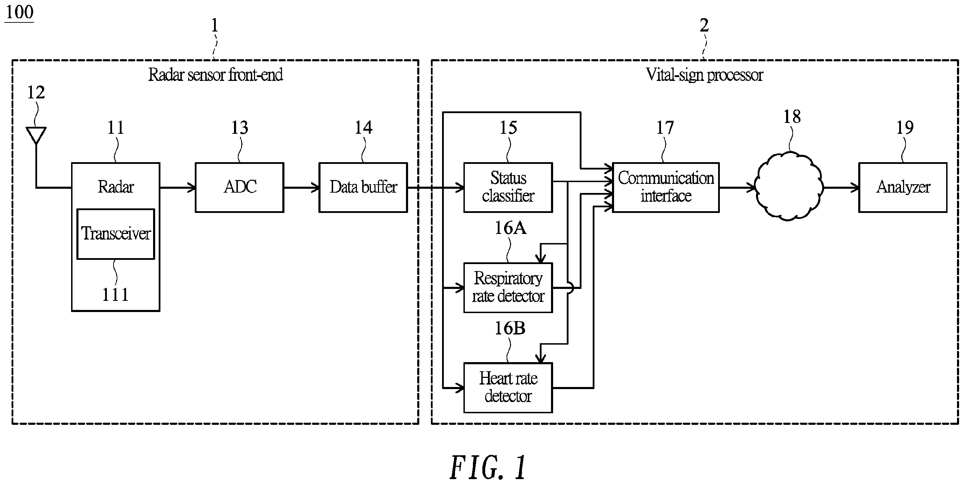

FIG. 1 shows a block diagram illustrating a non-contact vital-sign monitoring system 100 adaptable to monitoring a vital sign, such as a heart rate or a respiratory rate, according to one embodiment of the present invention.

In the embodiment, the non-contact vital-sign monitoring system (monitoring system hereinafter) 100 may include a radar sensor front-end 1 that may include a radar 11, such as a continuous-wave (CW) radar, disposed in vicinity of a monitored subject covered by the detection range of the radar 11. In one embodiment, the radar 11 may be disposed above the chest of the monitored subject lying on a bed. In another embodiment, the radar 11 may be disposed in other orientation such as below or beside the bed. In one embodiment, the radar 11 may include an ultra-wideband (UWB) radar such as frequency modulated continuous waveform (FMCW) radar. The radar sensor front-end 1 of the monitoring system 100 may include an antenna 12 electrically coupled to the radar 11, and configured to transmit radio-frequency (RF) signals and to receive reflected RF signals. The radar 11 of the embodiment may include a transceiver 111 configured to generate baseband output signals according to the reflected RF signals. In another embodiment, the antenna 12 may be integrated with the radar 11. In a further embodiment, the radar sensor front-end 1 may include multiple radars 11 disposed at different locations.

The radar sensor front-end 1 of the monitoring system 100 of the embodiment may include an analog-to-digital converter (ADC) 13 coupled to receive the (analog) output signals from the radar 11, and configured to convert the analog output signals into digital output signals. In the embodiment, the output signal of the radar 11 may include an in-phase polarization signal (in-phase signal hereinafter) I and a quadrature polarization signal (quadrature signal hereinafter) Q.

The radar sensor front-end 1 of the monitoring system 100 may include a data buffer 14 configured to store the output signals sampled in sequence during a predetermined period. For example, the data buffer 14 may store eight pieces of output signals sampled at intervals each of 2.5 seconds. Accordingly, the data buffer 14 may store the output signals spanning a period of 20 seconds. The spanning period, for example, of 10, 30 or 60 seconds may be set according to specific applications. The sampling interval may be determined based on response time. The shorter the sampling interval is, the more rapidly the monitoring system 100 responds.

In the embodiment, the monitoring system 100 may include a vital-sign processor 2 that may include a status classifier 15 configured to determine status of the monitored subject according to the output signals of the radar 11. In the embodiment, status of the monitored subject may, but not necessarily, be classified into three types: stationary, motion and no vital-sign. Stationary status may indicate that the monitored subject sleeps or rests, motion status may indicate that the monitored subject turns or moves, and no vital-sign status may indicate that the monitored subject is not currently on the bed. Specifically, for example, stationary status may indicate that the monitored subject turns to a posture during sleep, stays still while watching television, or slightly trembles; motion status may indicate that the monitored subject moves on bed or off bed, or moves around the bed; and no vital-sign status may indicate that the monitored subject is no longer vital. In another embodiment, the status classifier 15 may receive output signals of multiple radars 11 disposed at different locations.

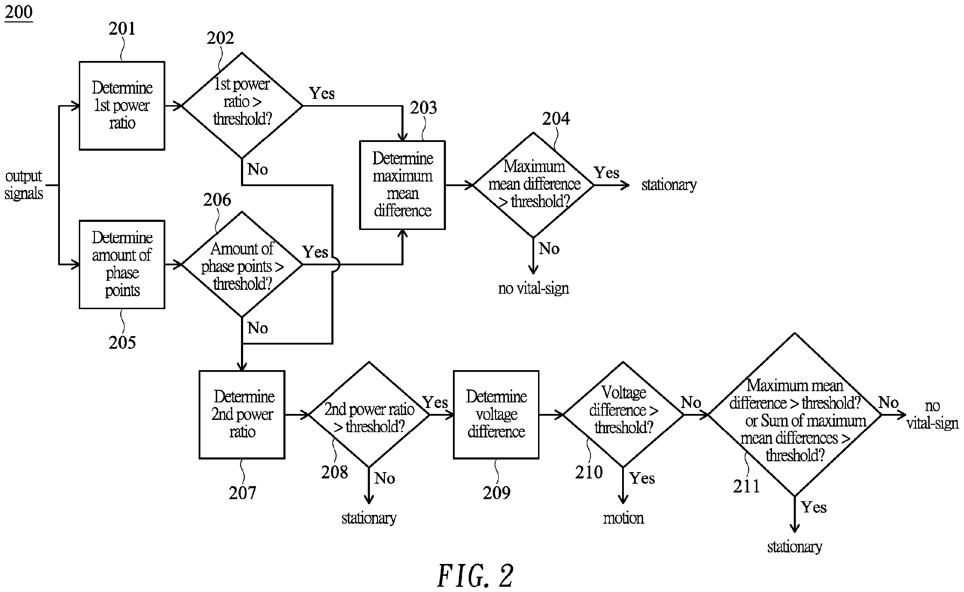

FIG. 2 shows a flow diagram illustrating a non-contact vital-sign monitoring method (method hereinafter) 200 for determining vital-sign status and adaptable to the status classifier 15 of FIG. 1 according to one embodiment of the present invention. In step 201, a first power ratio in frequency domain is determined according to the output signals of the radar 11, for example, by using fast Fourier transform (FFT) algorithm. In the embodiment, the first power ratio is defined as a ratio of power in a predetermined (first) frequency range (e.g., 12.5-25Hz) to total power, and may be expressed as:

first power ratio=$P_{fr1}/P_t$

where $P_{fr1}$ represents power in the predetermined (first) frequency range, and $P_t$ represents total power (in a full frequency range).

Next, in step 202, it is determined whether the first power ratio is greater than a predetermined (first) threshold.

If determination in step 202 is positive, the flow goes to step 203 to determine a maximum mean difference, which represents a maximum among a plurality of mean differences. In the embodiment, the data buffer 14 may have a total mean M. The data buffer 14 may be divided into a plurality of (e.g., m) blocks, each having a divisional mean $DM_1$-$DM_m$. The mean difference is defined as (the absolute value of) a difference of the divisional mean and the total mean, that is, $DM_x$-M, x is 1 to m. The maximum mean difference may be expressed as: maximum mean difference=max{abs[($DM_1$,$DM_2$,...$DM_m$)-(M,M,...M)]} where abs() represents absolute value function, and max() represents maximum value function.

In step 204, it is determined whether the maximum mean difference is greater than a predetermined (second) threshold. If determination in step 204 is positive, the monitored subject is determined or predicted as being in stationary status; otherwise the monitored subject is determined or predicted as being in no vital-sign status.

On the other hand, the method 200 for determining vital-sign status of the embodiment may include steps 205-206 to be executed concurrently with step 201-202. In step 205, an amount of phase point is determined according to the output signals of the radar 11. In the embodiment, the in-phase signal I, the quadrature signal Q and phase $\varphi$ may have the following relationship:

$$I(n)=A_I(n)\cos(p(n)+\theta)$$

$$Q(n)=A_Q(n)\sin(p(n)+\theta)$$

$$\varphi=\arctan[Q(n)/I(n)]$$

In the embodiment, if the phase $\varphi$ is within a predetermined range (e.g., between 44.5° and 45.5°), it is then numbered among the phase points. Next, in step 206, it is determined whether the amount of phase points is greater than a predetermined (third) threshold.

If determination in step 206 is positive, the flow goes to step 203 to determine a maximum mean difference. In step 204, it is determined whether the maximum mean difference is greater than a predetermined (second) threshold. If determination in step 204 is positive, the monitored subject is determined or predicted as being in stationary status; otherwise the monitored subject is determined or predicted as being in no vital-sign status.

If determination in step 202 or step 206 is negative, the flow goes to step 207 to determine a second power ratio in frequency domain according to the output signals of the radar 11, for example, by using fast Fourier transform (FFT) algorithm. In the embodiment, the second power ratio is defined as a ratio of power in a predetermined (second) frequency range (e.g., 3-25Hz) to total power, and may be expressed as:

$$\text{second power ratio} = P_{fr2}/P_t$$

where $P_{fr2}$ represents power in the predetermined (second) frequency range, and Pt represents total power (in a full frequency range). It is noted that the predetermined second frequency range may be equal to, or different from the predetermined first frequency range.

Next, in step 208, it is determined whether the second power ratio is greater than a predetermined (fourth) threshold, which may be equal to, or different from the predetermined (first) threshold. If determination in step 208 is negative, the monitored subject is determined or predicted as being in stationary status; otherwise the flow goes to step 209. If the predetermined second frequency range is equal to the predetermined first frequency range, and the predetermined fourth threshold is equal to the predetermined first threshold, steps 207-208 may be omitted for the reason that steps 207-208 are duplicates of steps 201-202.

In step 209, a voltage difference, such as point-to-point or peak-to-peak voltage difference, of the output signal is determined. Next, in step 210, it is determined whether the voltage difference is greater than a predetermined (fifth) threshold. If determination in step 210 is positive, the monitored subject is determined or predicted as being in motion status; otherwise the flow goes to step 211.

In step 211, it is determined whether the maximum mean difference is greater than a predetermined (second) threshold (similar to step 204), or whether a sum of maximum mean differences is greater than a predetermined (sixth) threshold. If at least one of determinations is positive, the monitored subject is determined or predicted as being in stationary status; otherwise the monitored subject is determined or predicted as being in no vital-sign status. In the embodiment, the sum of maximum mean differences is a sum of maximum mean difference of the in-phase signal I and maximum mean difference of the quadrature signal Q.

Referring back to FIG. 1, the vital-sign processor 2 of the monitoring system 100 of the embodiment may include a respiratory rate detector 16A coupled to receive the status determined by the status classifier 15 and configured to obtain a respiratory rate of the monitored subject according to the output signals (including the in-phase signal I and the quadrature signals Q) of the radar 11 of stationary status. As stated above, the data buffer 14 may store eight pieces of output signals sampled at intervals each of 2.5 seconds in the embodiment. While determining the respiratory rate, if the monitored subject is determined or predicted as being not in stationary status, corresponding data in the data buffer 14 may be set a predetermined value, such as a direct-current (DC) voltage value of the output signal. In another embodiment, the predetermined value may be a fixed waveform composed of one or more frequencies. In a further embodiment, the predetermined value may be a linear-nonlinear computation result according to preceding data stored in the data buffer 14.

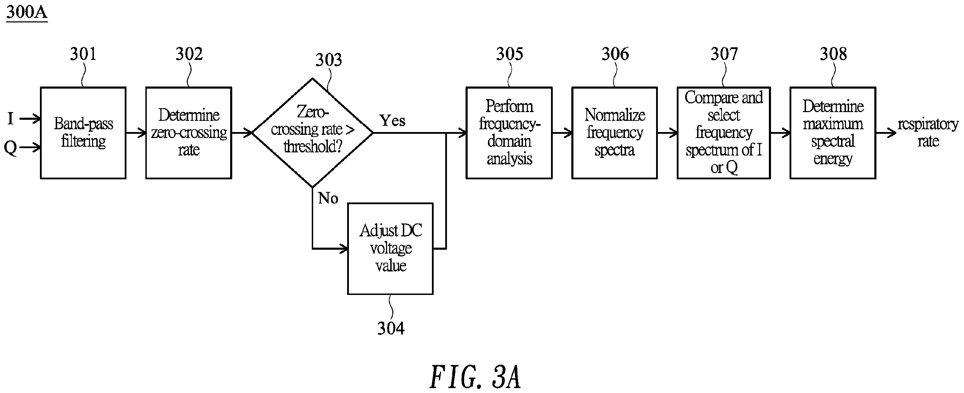

FIG. 3A shows a flow diagram illustrating a method 300A of detecting a respiratory rate adaptable to the respiratory rate detector 16A of FIG. 1 according to one embodiment of the present invention. In step 301, the output signals (including the in-phase signals I and the quadrature signals Q) of the radar 11 are subjected to band-pass filtering by a band-pass filter, thereby generating filtered signals. In one embodiment, a passband frequency range of the band-pass filter corresponds to a frequency range of respiratory rate, for example but not limited to, 0.16-0.8Hz.

In step 302, a zero-crossing rate in time domain is determined according to the filtered signals from the band-pass filter. In the embodiment, zero-crossing refers to an intersection between an alternative-current (AC) component of the output signal and a direct-current (DC) component of the output signal. When the output signal is normal, the zero-crossing rate is large. However, when the output signal is abnormal (e.g., the AC component shifts up or down), the zero-crossing rate becomes small.

Next, in step 303, it is determined whether the zero-crossing rate is greater than a predetermined (seventh) threshold. If determination in step 303 is negative (indicating that the AC component of the output signal shifts), the flow goes to step 304 to adjust a DC voltage value of the output signal.

If determination in step 303 is positive or step 304 finishes, the flow goes to step 305 to perform frequency-domain analysis, according to which a frequency spectrum (of energy distribution) of the in-phase signal I and a frequency spectrum (of energy distribution) of the quadrature signal Q are obtained. Next, in step 306, the frequency spectra of the in-phase signal I and the quadrature signal Q are normalized.

In step 307, a maximum spectral energy of the in-phase signal I and a maximum spectral energy of the quadrature signal Q are compared. Accordingly, the frequency spectrum of the in-phase signal I or the frequency spectrum of the quadrature signal Q is selected. In other words, the frequency spectrum of the in-phase signal I is selected if the maximum spectral energy of the in-phase signal I is greater than the maximum spectral energy of the quadrature signal Q; otherwise the frequency spectrum of the quadrature signal Q is selected. In step 308, a maximum spectral energy of the selected frequency spectrum is determined, and a corresponding frequency is determined as the respiratory rate.

Referring back to FIG. 1, the monitoring system 100 of the embodiment may include a heart rate detector 16B coupled to receive the status determined by the status classifier 15 and configured to obtain a heart rate of the monitored subject according to the output signals (including the in-phase signal I and the quadrature signals Q) of the radar 11 of stationary status.

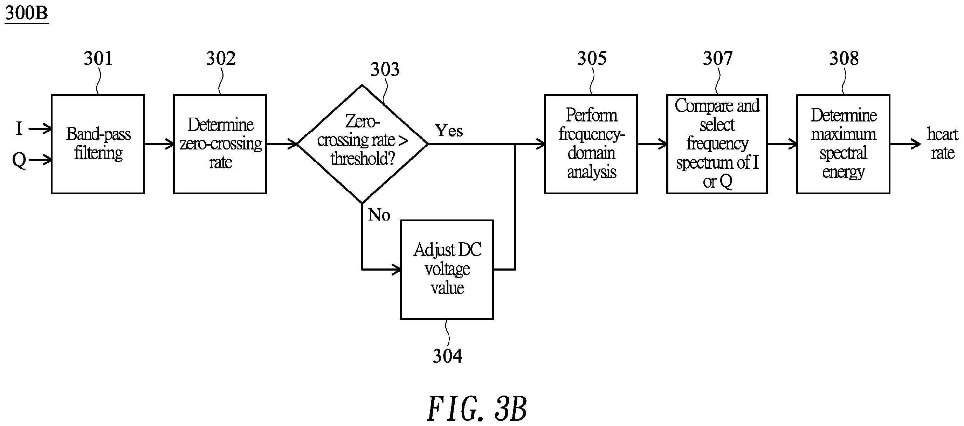

FIG. 3B shows a flow diagram illustrating a method 300B of detecting a heart rate adaptable to the heart rate detector 16B of FIG. 1 according to one embodiment of the present invention. The flow of FIG. 3B is similar to the flow of FIG. 3A, and corresponding steps are thus designated with the same numerals, difference of which will be described below.

In step 301, a passband frequency of the method 300B for detecting the heart rate is higher than (or equal to) the passband frequency of the method 300A for detecting the respiratory rate. In one embodiment, a passband frequency range of the method 300B for detecting the heart rate corresponds to a frequency range of heart rate, for example but not limited to, 0.7-3Hz. In the method 300B for detecting the heart rate, normalization on the frequency spectra in step 306 may be omitted. In step 308, a maximum spectral energy of the selected frequency spectrum is determined, and a corresponding frequency is determined as the heart rate.

Referring back to FIG. 1, the vital-sign processor 2 of the monitoring system 100 of the embodiment may include a communication interface 17 configured to transfer the output signals of the radar 11, the status determined by the status classifier 15, the respiratory rate of the monitored subject detected by the respiratory rate detector 16A and/or the heart rate of the monitored subject detected by the heart rate detector 16B to an analyzer 19 via a network 18 (e.g., the Internet). The communication interface 17 may be a wired communication interface such as universal asynchronous receiver-transmitter (UART), Inter-Integrated Circuit (I2C), Serial Peripheral Interface (SPI), Controller Area Network (CAN), Recommended Standard (RS) 232 or Recommended Standard (RS) 422; may be a wireless communication interface such as Wireless Sensor Network (e.g., EnOcean, Bluetooth or ZigBee); may be a cellular network such as second generation (2G), third generation (3G), Long-Term Evolution (LTE), or fifth generation (5G); may be a wireless local network such as WLAN or Worldwide Interoperability for Microwave Access (WiMAX); or may be a short range point-to-point communication such as Radio-frequency identification (RFID), EnOcean or Near-field communication. In another embodiment, the communication interface 17 is configured to transfer the output signals of the radar 11, the status determined by the status classifier 15, the respiratory rate of the monitored subject detected by the respiratory rate detector 16A and/or the heart rate of the monitored subject detected by the heart rate detector 16B to the analyzer 19 via a signal cable.

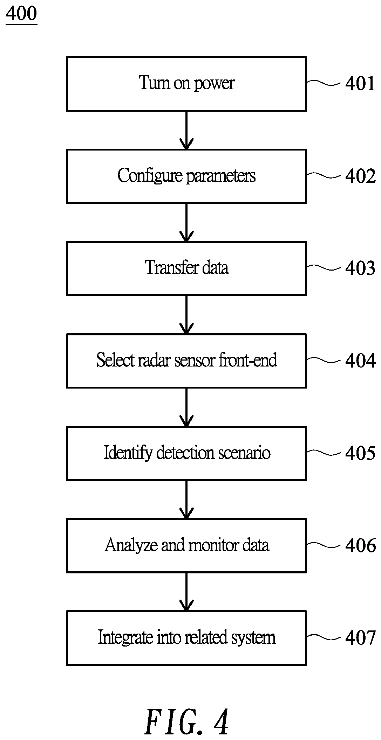

FIG. 4 shows a flow diagram illustrating a non-contact vital-sign monitoring method 400 according to one embodiment of the present invention. In step 401, power of the radar 11, the ADC 13, the data buffer 14, the status classifier 15, the respiratory rate detector 16A and the heart rate detector 16B is turned on. In the embodiment, the radar 11, the ADC 13, the data buffer 14, the status classifier 15, the respiratory rate detector 16A and the heart rate detector 16B may be implemented, for example, by a digital signal processor (DSP).

In step 402, primary parameters (e.g., division of the data buffer 14, the (first) frequency range (step 201), the (second) frequency range (step 207) and the passband frequency range (step 301)) of the data buffer 14, the status classifier 15, the respiratory rate detector 16A and the heart rate detector 16B are configured. In step 403, the communication interface 17 transfers the output signals, the status, the respiratory rate and/or the heart rate to the analyzer 19 (which may be disposed in cloud) via the network 18.

Next, in step 404, the analyzer 19 may select one among plural radar sensor front-ends 1. In step 405, a detection scenario (e.g., the monitored subject lies down on one's back, reclines, or lies down on one's side) is identified. In step 406, the transferred data of the radar sensor front-end 1 are analyzed and monitored. A warning may be issued to predetermined personnel or units when an emergent situation happens. In step 407, analysis result may be integrated into a related system (e.g., hospital system) for obtaining general and adequate judgement and comprehension.

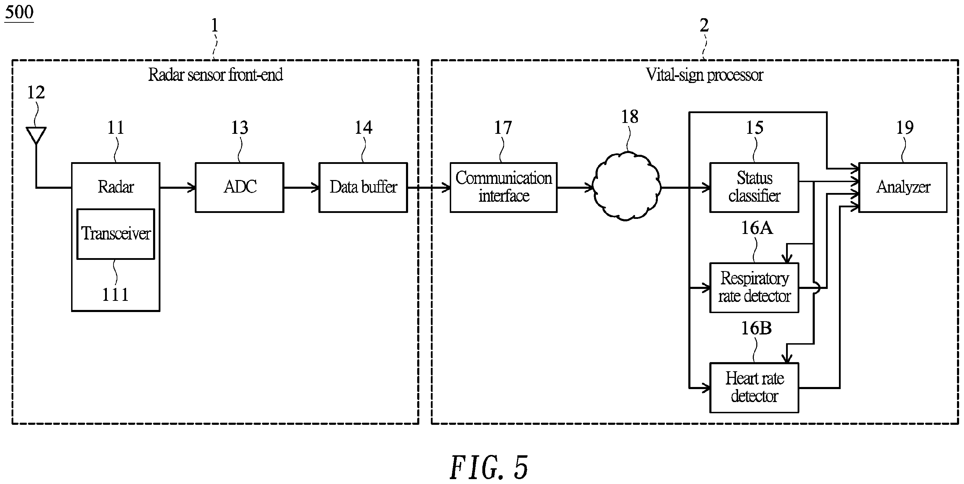

FIG. 5 shows a block diagram illustrating a non-contact vital-sign monitoring system (monitoring system hereinafter) 500 according to another embodiment of the present invention. The monitoring system 500 of FIG. 5 is similar to the monitoring system 100 of FIG. 1 with the following exception. In the embodiment, the communication interface 17 of the monitoring system 500 may receive the output signals of the radar 11 via the data buffer 14, and the output signals of the radar 11 may then be transferred to the status classifier 15, the respiratory rate detector 16A and/or the heart rate detector 16B via the network 18 (or directly). Accordingly, in the present embodiment, only the radar sensor front-end 1 (i.e., the radar 11, the antenna 12, the ADC 13 and the data buffer 14), but not the status classifier 15, the respiratory rate detector 16A and the heart rate detector 16B, need be installed in each ward.

**Claims**

1. A non-contact vital-sign status monitoring system (100) having a radar (11) disposed in vicinity of a monitored subject, the system comprising:

   a data buffer (14) storing buffered output signals of the radar sampled in sequence during a predetermined period;
   a status classifier (15) determining status of the monitored subject according to the buffered output signals of the radar; and
   a vital-sign detector (16A/16B) determining a vital sign of the monitored subject according to the buffered output signals of stationary status,

wherein the monitored subject is determined to be in a stationary status, a motion status or a no vital-sign status,

wherein the status classifier performs the following steps to determine the status of the monitored subjected:

determining a first power ratio (201) in frequency domain according to the buffered output signals of the radar, and determining whether the first power ratio is greater than a predetermined first threshold (202); determining an amount of phase points (205) according to the buffered output signals of the radar, and determining whether the amount of phase points is greater than a predetermined third threshold (206); determining a maximum mean difference (203) according to the buffered output signals of the radar if the first power ratio is greater than the first threshold and the amount of phase points is greater than the third threshold, and determining whether the maximum mean difference is greater than a predetermined second threshold (204);

wherein the first power ratio is defined as a ratio of power in a predetermined first frequency range to total power in a full frequency range; the amount of phase points is defined as an amount of the buffered output signals of the radar having a phase angle that is arctangent of a ratio of a quadrature signal to an in-phase signal within a predetermined range; the buffered output signals of the radar are divided into blocks each having a divisional mean, and the mean difference is defined as a difference between the divisional mean and a total mean of the entire buffered output signals of the radar; wherein the status classifier further performs the following steps to determine the status of the monitored subjected:

determining a second power ratio (207) in frequency domain according to the buffered output signals of the radar if the first power ratio is not greater than the first threshold or the amount of phase points is not greater than the third threshold, and determining whether the second power ratio is greater than a predetermined fourth threshold (208); and determining a voltage difference (209) according to the buffered output signals of the radar, and determining whether the voltage difference is greater than a predetermined fifth threshold (210);

wherein the second power ratio is defined as a ratio of power in a predetermined second frequency range to total power in the full frequency range; and the voltage difference is defined as point-to-point or peak-to-peak voltage difference of the buffered output signal of the radar;

wherein the monitored subject is determined as being in the stationary status if the first power ratio is not greater than the predetermined first threshold (202) and the second power ratio is not greater than the fourth threshold (208), or if the amount of the phase points is not greater than the predetermined third threshold (206) and the second power ratio is not greater than the fourth threshold (208).

2. The system of claim 1, wherein corresponding data in the data buffer is set a predetermined value when the monitored subject is not in stationary status.

3. The system of claim 1, wherein the vital-sign detector comprises a respiratory rate detector (16A), which receives the status determined by the status classifier and determines a respiratory rate of the monitored subject according to a frequency associated with a maximum spectral energy of the buffered output signals of stationary status.

4. The system of claim 1, wherein the vital-sign detector comprises a heart rate detector (16B), which receives the status determined by the status classifier and determines a heart rate of the monitored subject according to a frequency associated with a maximum spectral energy of the buffered output signals of stationary status.

5. A non-contact vital-sign status monitoring method (200), comprising:

determining a first power ratio (201) in frequency domain according to a plurality of buffered output signals of a radar (11) that is disposed in vicinity of a monitored subject, and determining whether the first power ratio is greater than a predetermined first threshold (202); determining an amount of phase points (205) according to the buffered output signals of the radar, and determining whether the amount of phase points is greater than a predetermined third threshold (206); determining a maximum mean difference (203) according to the buffered output signals of the radar if the first power ratio is greater than the first threshold and the amount of phase points is greater than the third threshold, and determining whether the maximum mean difference is greater than a predetermined second threshold (204);

determining a second power ratio (207) in frequency domain according to the buffered output signals of the radar if the first power ratio is not greater than the first threshold or the amount of phase points is not greater than the third threshold, and determining whether the second power ratio is greater than a predetermined fourth threshold (208); and

determining a voltage difference (209) according to the buffered output signals of the radar, and determining whether the voltage difference is greater than a predetermined fifth threshold (210);

wherein the monitored subject is determined to be in a stationary status, a motion status or a no vital-sign status,

wherein

the first power ratio is defined as a ratio of power in a predetermined first frequency range to total power in a full frequency range; the amount of phase points is defined as an amount of the buffered output signals of the radar having a phase angle that is arctangent of a ratio of a quadrature signal to an in-phase signal within a predetermined range; the buffered output signals of the radar are divided into blocks each having a divisional mean, and the mean difference is defined as a difference between the divisional mean and a total mean of the entire buffered output signals of the radar; the second power ratio is defined as a ratio of power in a predetermined second frequency range to total power in the full frequency range; and the voltage difference is defined as point-to-point or peak-to-peak voltage difference of the buffered output signal of the radar;

wherein the monitored subject is determined as being in the stationary status if the first power ratio is not greater than the predetermined first threshold (202) and the second power ratio is not greater than the fourth threshold (208), or if the amount of the phase points is not greater than the predetermined third threshold (206) and the second power ratio is not greater than the fourth threshold (208).

6.  The method of claim 5, wherein the monitored subject is also determined as being in the stationary status if the first power ratio is greater than the predetermined first threshold (202) or the amount of the phase points is greater than the predetermined third threshold (208), and if the maximum mean difference is greater than the second threshold (204), otherwise if the maximum mean difference is not greater than the second threshold (204) the monitored subject is determined as being in the no vital-sign status.

7.  The method of claim 5, wherein the monitored subject is determined as being in the motion status if the second power ratio is greater than the fourth threshold (208) and the voltage difference is greater than the fifth threshold (210).

8.  The method of claim 5, further comprising; if the second power ratio is determined to be greater than the fourth threshold (208) and the voltage difference is not greater than the fifth threshold (210), determining a sum of a maximum mean difference of the in-phase signal of the radar and a maximum mean difference of the quadrature signal of the radar, and determining whether the sum of the maximum mean difference of the in-phase signal of the radar and the maximum mean difference of the quadrature signal of the radar is greater than a predetermined sixth threshold (211).

9.  The method of claim 8, wherein the monitored subject is also determined as being in the stationary status if the voltage difference is not greater than the fifth threshold and the sum of the maximum mean difference of the in-phase signal of the radar and the maximum mean difference of the quadrature signal of the radar is greater than the sixth threshold, otherwise the monitored subject is determined as being in the no vital-sign status.

**Patentansprüche**

1.  System zum kontaktlosen Überwachen eines Vitalparameterzustands (100), das einen Radar (11) aufweist, der in der Nähe einer überwachten Person angeordnet ist, wobei das System umfasst:

einen Datenpuffer (14), der gepufferte Ausgangssignale des Radars speichert, die während eines vorbestimmten Zeitraums der Reihe nach abgetastet werden;
einen Zustandsklassifikator (15), der den Zustand der überwachten Person gemäß den gepufferten Ausgangssignalen des Radars bestimmt; und
einen Vitalparameterdetektor (16A/16B), der einen Vitalparameter der überwachten Person gemäß den gepufferten Ausgangssignalen des stationären Zustands bestimmt,
wobei bestimmt wird, dass sich die überwachte Person in einem stationären Zustand, einem Bewegungszustand oder einem vitalparameterlosen Zustand befindet,
wobei der Zustandsklassifikator die folgenden Schritte ausführt, um den Zustand der überwachten Person zu bestimmen:

Bestimmen eines ersten Leistungsverhältnisses (201) in der Frequenzdomäne gemäß den gepufferten Ausgangssignalen des Radars, und Bestimmen, ob das erste Leistungsverhältnis größer als eine vorbestimmte erste Schwelle (202) ist;
Bestimmen einer Menge von Phasenpunkten (205) gemäß den gepufferten Ausgangssignalen des Radars, und Bestimmen, ob die Menge von Phasenpunkten größer als eine vorbestimmte dritte Schwelle (206) ist;
Bestimmen einer maximalen Mittelwertdifferenz (203) gemäß den gepufferten Ausgangssignalen des Radars, falls das erste Leistungsverhältnis größer als die erste Schwelle ist und die Menge von Phasenpunkten größer als die dritte Schwelle ist, und Bestimmen, ob die maximale Mittelwertdifferenz größer als eine vorbestimmte zweite Schwelle (204) ist;
wobei das erste Leistungsverhältnis als ein Verhältnis der Leistung in einem vorbestimmten ersten Frequenzbereich zur Gesamtleistung in einem ganzen Frequenzbereich definiert ist; die Menge von Phasenpunkten als eine Menge der gepufferten Ausgangssignale des Radars definiert ist, der einen Phasenwinkel aufweist, welcher der Arkustangens eines Verhältnisses eines Phasenverschiebungssignals zu einem phasengleichen Signal innerhalb eines vorbestimmten Bereichs ist; die gepufferten Ausgangssignale des Radars in Blöcke unterteilt sind, die jeweils einen Teilungsmittelwert aufweisen, und die Mittelwertdifferenz als eine Differenz zwischen dem Teilungsmittelwert und einem Gesamtmittelwert der gesamten gepufferten Ausgangssignale des Radars definiert ist;
wobei der Zustandsklassifikator ferner die folgenden Schritte ausführt, um den Zustand der überwachten Person zu bestimmen:

Bestimmen eines zweiten Leistungsverhältnisses (207) in der Frequenzdomäne gemäß den gepufferten Ausgangssignalen des Radars, falls das erste Leistungsverhältnis nicht größer als die erste Schwelle ist oder die Menge von Phasenpunkten nicht größer als die dritte Schwelle ist, und Bestimmen, ob das zweite Leistungsverhältnis größer als eine vorbestimmte vierte Schwelle (208) ist; und
Bestimmen einer Spannungsdifferenz (209) gemäß den gepufferten Ausgangssignalen des Radars, und Bestimmen, ob die Spannungsdifferenz größer als eine vorbestimmte fünfte Schwelle (210) ist;
wobei das zweite Leistungsverhältnis als ein Verhältnis der Leistung in einem vorbestimmten zweiten Frequenzbereich zur Gesamtleistung in dem ganzen Frequenzbereich definiert ist; und die Spannungsdifferenz als Punkt-zu-Punkt- oder Spitze-zu-Spitze-Spannungsdifferenz des gepufferten Ausgangssignals des Radars definiert ist;
wobei bestimmt wird, dass sich die überwachte Person im stationären Zustand befindet, falls das erste Leistungsverhältnis nicht größer als die vorbestimmte erste Schwelle (202) ist und das zweite Leistungsverhältnis nicht größer als die vierte Schwelle (208)ist, oder falls die Menge der Phasenpunkte nicht größer als die vorbestimmte dritte Schwelle (206) ist und das zweite Leistungsverhältnis nicht größer als die vierte Schwelle (208) ist.

2. System nach Anspruch 1, wobei entsprechende Daten in dem Datenpuffer auf einen vorbestimmten Wert eingestellt werden, wenn sich die überwachte Person nicht im stationären Zustand befindet.

3. System nach Anspruch 1, wobei der Vitalparameterdetektor einen Atemfrequenzdetektor (16A) umfasst, der den Zustand empfängt, der durch den Zustandsklassifikator bestimmt wird, und eine Atemfrequenz der überwachten Person gemäß einer Frequenz bestimmt, die einer maximalen Spektralenergie der gepufferten Ausgangssignale des stationären Zustand zugeordnet ist.

4. System nach Anspruch 1, wobei der Vitalparameterdetektor einen Herzfrequenzdetektor (16B) umfasst, der den Zustand empfängt, der von dem Zustandsklassifikator bestimmt wird, und eine Herzfrequenz der überwachten Person gemäß einer Frequenz bestimmt, die einer maximalen Spektralenergie der gepufferten Ausgangssignale des stationären Zustand zugeordnet ist.

5. Verfahren (200) zum kontaktlosen Überwachen eines Vitalparameterzustands, umfassend:

Bestimmen eines ersten Leistungsverhältnisses (201) in der Frequenzdomäne gemäß einer Vielzahl von gepufferten Ausgangssignalen eines Radars (11), der in der Nähe einer überwachten Person angeordnet ist, und Bestimmen, ob das erste Leistungsverhältnis größer als eine vorbestimmte erste Schwelle (202) ist; Bestimmen einer Menge von Phasenpunkten (205) gemäß den gepufferten Ausgangssignalen des Radars, und Bestimmen, ob die Menge von Phasenpunkten größer als eine vorbestimmte dritte Schwelle (206) ist; Bestimmen einer maximalen Mittelwertdifferenz (203) gemäß den gepufferten Ausgangssignalen des Radars, falls das erste Leistungsverhältnis größer als die erste Schwelle ist und die Menge von Phasenpunkten größer als die dritte Schwelle ist, und Bestimmen, ob die maximale Mittelwertdifferenz größer als eine vorbestimmte zweite Schwelle (204) ist; Bestimmen eines zweiten Leistungsverhältnisses (207) in der Frequenzdomäne gemäß den gepufferten Ausgangssignalen des Radars, falls das erste Leistungsverhältnis nicht größer als die erste Schwelle ist oder die Menge von Phasenpunkten nicht größer als die dritte Schwelle ist, und Bestimmen, ob das zweite Leistungsverhältnis größer als eine vorbestimmte vierte Schwelle (208) ist; und Bestimmen einer Spannungsdifferenz (209) gemäß den gepufferten Ausgangssignalen des Radars, und Bestimmen, ob die Spannungsdifferenz größer als eine vorbestimmte fünfte Schwelle (210) ist; wobei bestimmt wird, dass sich die überwachte Person in einem stationären Zustand, einem Bewegungszustand oder einem vitalparameterlosen Zustand befindet, wobei das erste Leistungsverhältnis als ein Verhältnis der Leistung in einem vorbestimmten ersten Frequenzbereich zur Gesamtleistung in einem ganzen Frequenzbereich definiert ist; die Menge von Phasenpunkten als eine Menge der gepufferten Ausgangssignale des Radars definiert ist, der einen Phasenwinkel aufweist, welcher der Arkustangens eines Verhältnisses eines Phasenverschiebungssignals zu einem phasengleichen Signal innerhalb eines vorbestimmten Bereichs ist; die gepufferten Ausgangssignale des Radars in Blöcke unterteilt sind, die jeweils einen Teilungsmittelwert aufweisen, und die Mittelwertdifferenz als eine Differenz zwischen dem Teilungsmittelwert und einem Gesamtmittelwert der gesamten gepufferten Ausgangssignale des Radars definiert ist; das zweite Leistungsverhältnis als ein Verhältnis der Leistung in einem vorbestimmten zweiten Frequenzbereich zur Gesamtleistung in einem ganzen Frequenzbereich definiert ist; und die Spannungsdifferenz als Punkt-zu-Punkt- oder Spitze-zu-Spitze-Spannungsdifferenz des gepufferten Ausgangssignals des Radars definiert ist; wobei bestimmt wird, dass sich die überwachte Person im stationären Zustand befindet, falls das erste Leistungsverhältnis nicht größer als die vorbestimmte erste Schwelle (202) ist und das zweite Leistungsverhältnis nicht größer als die vierte Schwelle (208) ist, oder falls die Menge der Phasenpunkte nicht größer als die vorbestimmte dritte Schwelle (206) ist und das zweite Leistungsverhältnis nicht größer als die vierte Schwelle (208) ist.

6. Verfahren nach Anspruch 5, wobei ebenfalls bestimmt wird, dass sich die überwachte Person im stationären Zustand befindet, falls das erste Leistungsverhältnis größer als die vorbestimmte erste Schwelle (202) ist oder die Menge der Phasenpunkte größer als die vorbestimmte dritte Schwelle (208) ist, und falls die maximale Mittelwertdifferenz größer als die zweite Schwelle (204) ist, ansonsten falls die maximale Mittelwertdifferenz nicht größer als die zweite Schwelle (204) ist, wird bestimmt, dass sich die überwachte Person in einem vitalparameterlosen Zustand befindet.

7. Verfahren nach Anspruch 5, wobei bestimmt wird, dass sich die überwachte Person im Bewegungszustand befindet, falls das zweite Leistungsverhältnis größer als die vierte Schwelle (208) ist und die Spannungsdifferenz größer als die fünfte Schwelle (210) ist.

8. Verfahren nach Anspruch 5, ferner umfassend;

   falls bestimmt wird, dass das zweite Leistungsverhältnis größer als die vierte Schwelle (208) ist und die Spannungsdifferenz nicht größer als die fünfte Schwelle (210) ist, Bestimmen einer Summe einer maximalen Mittelwertdifferenz des phasengleichen Signals des Radars und einer maximalen Mittelwertdifferenz des Phasenverschiebungssignals des Radars, und Bestimmen, ob die Summe der maximalen Mittelwertdifferenz des phasengleichen Signals des Radars und der maximalen Mittelwertdifferenz des Phasenverschiebungssignals des Radars größer als eine vorbestimmte sechste Schwelle (211) ist.

9. Verfahren nach Anspruch 8, wobei ebenfalls bestimmt wird, dass sich die überwachte Person im stationären Zustand befindet, falls die Spannungsdifferenz nicht größer als die fünfte Schwelle ist und die Summe der maximalen Mittelwertdifferenz des phasengleichen Signals des Radars und der maximalen Mittelwertdifferenz des Phasenverschiebungssignals des Radars größer als die sechste Schwelle ist, wobei ansonsten bestimmt wird, dass sich die überwachte Person im vitalparameterlosen Zustand befindet.

## Revendications

1. Système de surveillance d'état de signe vital sans contact (100) comportant un radar (11) disposé à proximité d'un sujet surveillé, le système comprenant :

   une mémoire tampon de données (14) stockant des signaux de sortie mis en mémoire tampon du radar échantillonnés en séquence au cours d'une période prédéterminée ;
   un classificateur d'état (15) déterminant un état du sujet surveillé en fonction des signaux de sortie mis en mémoire tampon du radar ; et
   un détecteur de signe vital (16A/16B) déterminant un signe vital du sujet surveillé en fonction des signaux de sortie mis en mémoire tampon d'état immobile,
   dans lequel il est déterminé que le sujet surveillé est dans un état immobile, un état mobile ou un état sans signe vital,
   dans lequel
   le classificateur d'état réalise les étapes suivantes pour déterminer l'état du sujet surveillé :

      la détermination d'un premier rapport de puissance (201) dans un domaine fréquentiel en fonction des signaux de sortie mis en mémoire tampon du radar, et la détermination si le premier rapport de puissance est supérieur à un premier seuil prédéterminé (202) ;
      la détermination d'une quantité de points de phase (205) en fonction des signaux de sortie mis en mémoire tampon du radar, et la détermination si la quantité de points de phase est supérieure à un troisième seuil prédéterminé (206) ;
      la détermination d'une différence de moyenne maximale (203) en fonction des signaux de sortie mis en mémoire tampon du radar si le premier rapport de puissance est supérieur au premier seuil et la quantité de points de phase est supérieure au troisième seuil, et la détermination si la différence de moyenne maximale est supérieure à un deuxième seuil prédéterminé (204) ;
      dans lequel le premier rapport de puissance est défini en tant qu'un rapport d'une puissance dans une première plage de fréquence prédéterminée sur une puissance totale dans une plage de fréquence complète ; la quantité de points de phase est définie en tant qu'une quantité des signaux de sortie mis en mémoire tampon du radar présentant un angle de phase qui est l'arctangente d'un rapport d'un signal en quadrature sur un signal en phase à l'intérieur d'une plage prédéterminée ; les signaux de sortie mis en mémoire tampon du radar sont divisés en blocs comportant chacun une moyenne divisionnelle, et la différence de moyenne est définie en tant qu'une différence entre la moyenne divisionnelle et une moyenne totale de tous les signaux de sortie mis en mémoire tampon du radar ;
      dans lequel le classificateur d'état réalise en outre les étapes suivantes pour déterminer l'état du sujet surveillé :

         la détermination d'un deuxième rapport de puissance (207) dans un domaine fréquentiel en fonction des signaux de sortie mis en mémoire tampon du radar si le premier rapport de puissance n'est pas supérieur au premier seuil ou la quantité de points de phase n'est pas supérieure au troisième seuil, et la détermination si le deuxième rapport de puissance est supérieur à un quatrième seuil prédéterminé (208) ; et
         la détermination d'une différence de tension (209) en fonction des signaux de sortie mis en mémoire tampon du radar, et la détermination si la différence de tension est supérieure à un cinquième seuil prédéterminé (210) ;
         dans lequel le deuxième rapport de puissance est défini en tant qu'un rapport d'une puissance dans une deuxième plage de fréquence prédéterminée sur une puissance totale dans la plage de fréquence complète ; et la différence de tension est définie en tant qu'une différence de tension de point à point ou de crête à crête du signal de sortie mis en mémoire tampon du radar ;
         dans lequel il est déterminé que le sujet surveillé est dans l'état immobile si le premier rapport de puissance n'est pas supérieur au premier seuil prédéterminé (202) et le deuxième rapport de puissance n'est pas supérieur au quatrième seuil (208), ou si la quantité des points de phase n'est pas supérieure au troisième seuil prédéterminé (206) et le deuxième rapport de puissance n'est pas supérieur au quatrième seuil (208).

**2.** Système selon la revendication 1, dans lequel une valeur prédéterminée est attribuée à des données correspondantes dans la mémoire tampon de données lorsque le sujet surveillé n'est pas dans un état immobile.

**3.** Système selon la revendication 1, dans lequel le détecteur de signe vital comprend un détecteur de fréquence respiratoire (16A), qui reçoit l'état déterminé par le classificateur d'état et détermine une fréquence respiratoire du sujet surveillé en fonction d'une fréquence associée à une énergie spectrale maximale des signaux de sortie mis en mémoire tampon d'état immobile.

**4.** Système selon la revendication 1, dans lequel le détecteur de signe vital comprend un détecteur de fréquence cardiaque (16B), qui reçoit l'état déterminé par le classificateur d'état et détermine une fréquence cardiaque du sujet surveillé en fonction d'une fréquence associée à une énergie spectrale maximale des signaux de sortie mis en mémoire tampon d'état immobile.

**5.** Procédé de surveillance d'état de signe vital sans contact (200), comprenant :

la détermination d'un premier rapport de puissance (201) dans un domaine fréquentiel en fonction d'une pluralité de signaux de sortie mis en mémoire tampon d'un radar (11) qui est disposé à proximité d'un sujet surveillé, et la détermination si le premier rapport de puissance est supérieur à un premier seuil prédéterminé (202) ;

la détermination d'une quantité de points de phase (205) en fonction des signaux de sortie mis en mémoire tampon du radar, et la détermination si la quantité de points de phase est supérieure à un troisième seuil prédéterminé (206) ;

la détermination d'une différence de moyenne maximale (203) en fonction des signaux de sortie mis en mémoire tampon du radar si le premier rapport de puissance est supérieur au premier seuil et la quantité de points de phase est supérieure au troisième seuil, et la détermination si la différence de moyenne maximale est supérieure à un deuxième seuil prédéterminé (204) ;

la détermination d'un deuxième rapport de puissance (207) dans un domaine fréquentiel en fonction des signaux de sortie mis en mémoire tampon du radar si le premier rapport de puissance n'est pas supérieur au premier seuil ou la quantité de points de phase n'est pas supérieure au troisième seuil, et la détermination si le deuxième rapport de puissance est supérieur à un quatrième seuil prédéterminé (208) ; et

la détermination d'une différence de tension (209) en fonction des signaux de sortie mis en mémoire tampon du radar, et la détermination si la différence de tension est supérieure à un cinquième seuil prédéterminé (210) ;

dans lequel il est déterminé que le sujet surveillé est dans un état immobile, un état mobile ou un état sans signe vital,

dans lequel

le premier rapport de puissance est défini en tant qu'un rapport d'une puissance dans une première plage de fréquence prédéterminée sur une puissance totale dans une plage de fréquence complète ; la quantité de points de phase est définie en tant qu'une quantité des signaux de sortie mis en mémoire tampon du radar présentant un angle de phase qui est l'arctangente d'un rapport d'un signal en quadrature sur un signal en phase à l'intérieur d'une plage prédéterminée ; les signaux de sortie mis en mémoire tampon du radar sont divisés en blocs comportant chacun une moyenne divisionnelle, et la différence de moyenne est définie en tant qu'une différence entre la moyenne divisionnelle et une moyenne totale de tous les signaux de sortie mis en mémoire tampon du radar ; le deuxième rapport de puissance est défini en tant qu'un rapport d'une puissance dans une deuxième plage de fréquence prédéterminée sur une puissance totale dans la plage de fréquence complète ; et la différence de tension est définie en tant qu'une différence de tension de point à point ou de crête à crête du signal de sortie mis en mémoire tampon du radar ;

dans lequel il est déterminé que le sujet surveillé est dans l'état immobile si le premier rapport de puissance n'est pas supérieur au premier seuil prédéterminé (202) et le deuxième rapport de puissance n'est pas supérieur au quatrième seuil (208), ou si la quantité des points de phase n'est pas supérieure au troisième seuil prédéterminé (206) et le deuxième rapport de puissance n'est pas supérieur au quatrième seuil (208).

**6.** Procédé selon la revendication 5, dans lequel il est également déterminé que le sujet surveillé est dans l'état immobile si le premier rapport de puissance est supérieur au premier seuil prédéterminé (202) ou la quantité des points de phase est supérieure au troisième seuil prédéterminé (208), et si la différence de moyenne maximale est supérieure au deuxième seuil (204) sinon, si la différence de moyenne maximale n'est pas supérieure au deuxième seuil (204), il est déterminé que le sujet surveillé est dans l'état sans signe vital.

**7.** Procédé selon la revendication 5, dans lequel il est déterminé que le sujet surveillé est dans l'état mobile si le deuxième rapport de puissance est supérieur au quatrième seuil (208) et la différence de tension est supérieure au cinquième seuil (210).

8. Procédé selon la revendication 5, comprenant en outre : s'il est déterminé que le deuxième rapport de puissance est supérieur au quatrième seuil (208) et la différence de tension n'est pas supérieure au cinquième seuil (210), la détermination d'une somme d'une différence de moyenne maximale du signal en phase du radar et d'une différence de moyenne maximale du signal en quadrature du radar, et la détermination si la somme de la différence de moyenne maximale du signal en phase du radar et de la différence de moyenne maximale du signal en quadrature du radar est supérieure à un sixième seuil prédéterminé (211).

9. Procédé selon la revendication 8, dans lequel il est également déterminé que le sujet surveillé est dans l'état immobile si la différence de tension n'est pas supérieure au cinquième seuil et la somme de la différence de moyenne maximale du signal en phase du radar et de la somme de moyenne maximale du signal en quadrature du radar est supérieure au sixième seuil, sinon il est déterminé que le sujet surveillé est dans l'état sans signe vital.

FIG. 1

**FIG. 2**

EP 3 671 260 B1

300A

I →
Q →

**301** Band-pass filtering

**302** Determine zero-crossing rate

**303** Zero-crossing rate > threshold?

No → **304** Adjust DC voltage value

Yes →

**305** Perform frequency-domain analysis

**306** Normalize frequency spectra

**307** Compare and select frequency spectrum of I or Q

**308** Determine maximum spectral energy

→ respiratory rate

*FIG. 3A*

300B

*FIG. 3B*

EP 3 671 260 B1

<u>400</u>

```
┌─────────────────────────────┐
│      Turn on power          │──── 401
└─────────────────────────────┘
              │
              ▼
┌─────────────────────────────┐
│     Configure parameters    │──── 402
└─────────────────────────────┘
              │
              ▼
┌─────────────────────────────┐
│        Transfer data        │──── 403
└─────────────────────────────┘
              │
              ▼
┌─────────────────────────────┐
│   Select radar sensor front-end │──── 404
└─────────────────────────────┘
              │
              ▼
┌─────────────────────────────┐
│   Identify detection scenario   │──── 405
└─────────────────────────────┘
              │
              ▼
┌─────────────────────────────┐
│     Analyze and monitor data    │──── 406
└─────────────────────────────┘
              │
              ▼
┌─────────────────────────────┐
│   Integrate into related system │──── 407
└─────────────────────────────┘
```

*FIG. 4*

FIG. 5

EP 3 671 260 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007101343 A1